# FASCICULE DE BREVET EUROPEEN

(11) **EP 0 581 980 B1**
(45) Date de publication et mention de la délivrance du brevet: **03.03.1999**
(21) Numéro de dépôt: 92113190.0
(22) Date de dépôt: 03.08.1992
(51) Int. Cl.: C07D 307/00, C07C 67/29

(54) **Dérivés de l'acide quinique 3- et/ou 4-substitués et procédé de préparation de dérivés de l'acide quinique 3- et/ou 4-substitués**
3- und/oder 4-substituierte Chinasäurederivate und Verfahren zur Herstellung von 3- und/oder 4-substituierten Chinasäurederivaten
3- and/or 4-substituted derivatives of quinic acid and process to prepare 3- and/or 4-substituted derivatives of quinic acid

(43) Date de publication de la demande: 09.02.1994
(73) Titulaire: SOCIETE DES PRODUITS NESTLE S.A., 1800 Vevey (CH)
(72) Inventeur: Huynh-Ba, Tuong, CH-1009 Pully (CH)
(74) Mandataire: Archambault, Jean

(56) Documents cités:
- WO-A-86/01508
- GB-A- 802 668
- CHEMICAL ABSTRACTS, vol. 116, no. 23, 8 Juin 1992, Columbus, Ohio, US; abstract no. 236095z, page 880 ;

## Description

L'invention concerne de nouveaux dérivés de l'acide quinique 3- et/ou 4-substitués et un procédé de préparation de ces dérivés.

Les dérivés de l'acide quinique sont largement répandus dans le monde végétal. Le contenu en acide chlorogénique des fèves de café vert, par exemple, peut représenter jusque 10% de la matière sèche. Le terme "acide chlorogénique" recouvre en fait un mélange relativement complexe de dérivés de l'acide quinique parmi lesquels on peut citer les acides caféoyl-, dicaféoyl-, p-coumaroyl-, féruloyl- et caféoylféruloyl-quinique. Après torréfaction, les grains de café contiennent un mélange de composés encore plus complexe dont certains sont constitués de lactones des acides quiniques appelées quinides ou encore d'autres isomères formés lors du traitement thermique. Certains de ces dérivés possèdent une activité antimicrobienne, antagoniste des opiacés, antiinflammatoire, fongistatique, cytostatique et inhibitrice de la biosynthèse des leucotriènes. Nous avons trouvé que certains de ces composés possédaient une activité anti-uréase dont l'application fait l'objet de la demande de brevet parallèle de la titulaire déposée sous le titre "composition cosmétique ou dermatologique anti-uréase".

La synthèse des acides hydroxycinnamoyl-quiniques est décrite par exemple dans J. Chem. Soc. 1964, 2137-2146, E. Haslam et col.. Cependant, les procédés connus requièrent une étape de séparation délicate et laborieuse basée sur la technique de distribution à contre-courant en vue d'isoler le composé cherché dans la mesure où l'étape clé d'estérification n'est pas régiosélective et conduit à un mélange d'isomères qu'il faut séparer. De plus, la méthode connue fait usage de groupes protecteurs des réactifs hydroxycinnamiques qui ne peuvent être clivés que dans des conditions drastiques (acide fort ou base forte et chaleur) qui provoquent l'isomérisation des dérivés chlorogéniques.

Le brevet WO-A-86/01508 décrit la synthèse d'antagonistes des opiacés, en particulier des quinides 1, 3 ou 4 monosubstituées. Dans ce document, le choix des groupes de protection et des méthodes de clivage présente des inconvénients non négligeables. Les conditions de clivage, par un traitement avec K2CO3 dans du méchanol aqueux, sont telles que le risque d'ouverture partielle de la lactone et d'isomérisation du groupe féruloyl ou isoféruloyl de la position 1 et positions 3, 4 et 5 devient non négligeable, une purification ultérieure devient nécessaire, ce qui diminue d'autant le rendement de la réaction.

Le document GB-A-802668 décrit des esters caféiques de l'acide quinique et de la quinide, en particulier la 1-O-caféoyl quinide, sa préparation et la synthèse des acides 1-O-caféoyl et 1,4-O-dicaféoyl quiniques par hydrolyse des quinides correspondantes. Cette méthode de préparation est susceptible de provoquer des réactions d'isomérisation partielle du groupe caféoyl en positions 4 et 5, d'oxydation du groupe catéchol de l'acide quinique et de polymérisation. De plus, un tel procédé n'est pas sélectif, ce qui conduit inévitablement à un mélange de composés.

L'article CA 116, 23, 236095z, décrit la synthèse de l'acide 4,5 bis-(3,4-diméthoxycinnamoyl) quinique. Le choix dans les conditions de préparation dudit acide quinique à partir de la quinide correspondante a l'inconvénient de présenter un risque d'isomérisation du groupe 3,4-diméthoxycinnamoyl des positions 4 et 5 en positions 1 et 3 du squelette d'acide quinique qui n'est pas négligeable.

L'invention concerne des quinides destinées à servir de composés intermédiaires dans la synthèse d'acides quiniques 3- ou 4-mono-, 1,3-, 1,4- ou 3,4-disubstitués, de formule : 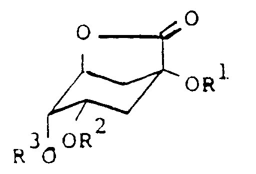 dans laquelle R1 est H et l'un des deux groupes R2 et R3 représente un groupe p-coumaroyl et l'autre est H, ou les deux groupes R2 et R3 représentent un groupe p-coumaroyl, p-feruloyl ou p-caféoyl, R2 étant différent de R3, ou R1 est un groupe p-coumaroyl, p-feruloyl ou p-caféoyl et R2 est H et R3 représente un groupe p-coumaroyl ou p-feruloyl ou R2 est un groupe p-coumaroyl, p-feruloyl ou p-caféoyl et R3 est H.
Les nouveaux composés selon l'invention constituent des produits intermédiaires très utiles dans la préparation des acides quiniques ayant les applications intéressantes mentionnées précédemment.

L'invention concerne également un procédé de préparation de dérivés de l'acide quinique 3- ou 4-mono-, 1,3-, 1,4-ou 3,4-disubstitué, dans lequel le substituant en position 3 est différent du substituant en position 4 et dans lequel on fait réagir un dérivé d'un acide hydroxycinnamique dont le ou les groupes hydroxyles sont protégés, avec un dérivé de l'acide quinique protégé, de manière à former un ester, puis on clive les groupes protecteurs.
Ce procédé est caractérisé par le fait que le dérivé de l'acide quinique mis en oeuvre est une quinide, que et que le dérivé d'acide quinique final est obtenu par hydrolyse de la quinide dans des conditions ménagées, que l'on protège les fonctions OH en position 3, 4 et 5 et la fonction carboxylique en position 1 de l'acide quinique par réaction avec l'acétone conduisant à la 3,4-O-isopropylidène quinide, puis on introduit un groupe protecteur carbonate en position 1, que l'on libère les fonctions OH en positions 3 et 4, que l'on estérifie sélectivement la position 3 en conduisant l'estérification à une température inférieure à - 10°C avec un dérivé réactif d'un acide hydroxycinnamique dont la ou les fonctions phénol ont été préalablement protégées par des groupes carbonates, que l'on libère le groupe protecteur acétonide, puis le ou les groupes protecteurs carbonates dans des conditions ménagées d'acidité et de température, les dites conditions ménagées permettant d'éviter toute dégradation ou isomérisation lors de la déprotection.

Dans un premier mode de réalisation du procédé selon l'invention, on prépare avec un bon rendement des dérivés monosubstitués 3-O-hydroxycinnamoyl de la quinide que l'on transforme ensuite en l'acide quinique. Pour ce faire, on protège les fonctions OH en position 3, 4 et 5 et la fonction carboxylique en position 1 de l'acide quinique, par exemple par réaction avec l'acétone en présence d'acide paratoluènesulfonique, ce qui conduit à la 3,4-O-isopropylidène-quinide.
On introduit alors un goupe protecteur en position 1 qui est un carbonate, par exemple le trichloroéthyl carbonate, qui peut être clivé dans des conditions modérées, par exemple par le zinc en milieu acétique à la température ambiante.
Avant le clivage du carbonate protecteur en position 1, il est nécessaire de libérer le groupe acétonide protecteur des fonctions OH en position 3 et 4 de la quinide. Pour ce faire, on utilise une solution aqueuse très concentrée d'acide trifluoroacétique à la température ambiante.
Une fois les positions 3 et 4 libérées, on procède à l'estérification sélective de la position 3 en tirant parti du fait que le groupe OH en position 3 a une conformation équatoriale dont la substitution est plus stable que celle du groupe OH en position 4 de configuration axiale, pour autant que l'on contrôle correctement l'énergie de la réaction. Il est ainsi possible d'introduire sélectivement un substituant p-coumaryl ou féruloyl à partir d'un dérivé réactif de l'acide correspondant tel que, par exemple le chlorure ou l'anhydride en conduisant la réaction à une température < à -10°C, de préférence à environ -40°C. En effet, nous avons remarqué qu'à une température > à -10°C l'ester en position 4 se forme de manière concomitante. Après clivage du carbonate protecteur de la position 1, dans des conditions ménagées, par exemple par le zinc dans l'acide acétique à la température ambiante, on obtient les quinides 3 substituées. Les conditions ménagées précédentes permettent d'éviter toute décomposition qui interviendrait dans les conditions classiques d'utilisation de l'acide acétique à haute température.
Selon une variante de ce mode de réalisation, destinée à préparer les dérivés 3-caféoyl substitués, on utilise le méthyl carbonate comme groupe protecteur des fonctions phénol de l'acide caféique. Après réaction, on clive successivement le groupe protecteur trichloroéthyl carbonate par le zinc en présence d'acide acétique, puis les groupes méthyl carbonate par le chlorure de lithium dans la pyridine en présence de chlorotriméthylsilane. Le chlorotriméthylsilane a pour fonction de prévenir la migration du radical caféoyl de la position 3 à la position 4 du squelette de la'acide quinique.
Enfin, pour préparer les acides quiniques correspondant aux quinides, on procède à l'hydrolyse acide de ces dernières, également dans des conditions ménagées, par exemple par une quantité catalytique d'acide p-toluènesulfonique dans le cas des dérivés 3-p-coumaroyl et 3-féruloyl.
Dans le cas des dérivés 3-caféoyl, l'ouverture du cycle lactone de la quinide, par exemple par l'acide chlorhydrique aqueux en présence d'un solvant polaire, par exemple le tétrahydrofurane, ne peut se faire qu'après avoir complétement protégé tous les groupes hydroxy, par exemple par des groupes trichloroéthyl carbonate qui peuvent ensuite être clivés, par exemple par le zinc dans l'acide acétique.

Dans un second mode de réalisation du procédé selon l'invention, pour préparer les dérivés 4-monosubstitués de l'acide quinique, on tire profit de la méthode précédente d'estérification sélective de la quinide en position 3 en introduisant dans cette position un groupe protecteur trichloroéthyl carbonate.

On utilise ensuite la quinide protégée comme matière première d'estérification de la seule fonction OH encore libre, en position 4, par un dérivé réactif, par exemple un anhydride ou un chlorure d'acide dont les fonctions phénol ont été préalablement également protégées comme indiqué précédemment.
On procède ensuite au clivage successif des différents groupes protecteurs comme indiqué précédemment et le cas échéant à l'hydrolyse acide de la quinide en l'acide quinique correspondant.
Pour cette dernière hydrolyse, on doit veiller à ce qu'il ne se produise pas d'isomérisation des acides acyl quiniques entre les positions 3 et 4, auquel cas tout le bénéfice de la sélectivité de l'estérification en position 3 serait perdu. Pour l'éviter, on réalise l'hydrolyse du cycle lactone de la quinide entièrement protégée préalablement au clivage des groupes protecteurs. Dans le cas du substituant caféoyl, la protection de ses fonctions phénol ne s'est pas avérée nécessaire puisque l'ouverture du cycle lactone de la quinide n'a pas donné lieu à une quelconque isomérisation.

Dans un troisième mode de réalisation du procédé de l'invention, on prépare les dérivés 1,3- et 1,4-disubstitués de l'acide quinique toujours en tirant parti de l'estérification sélective de la fonction OH en position 3 du squelette de la quinide et, dans le cas de la préparation des acides quiniques correspondants, en contrôlant l'isomérisation intervenant entre les positions 3 et 4 des acides caféoyl quiniques.
Pour la préparation des dérivés 1,3-O-dicaféoyl on utilise de préférence un dérivé réactif de l'acide dicarbométhoxy caféique, par exemple le chlorure d'acide dans l'étape d'estérification sélective en position 3, car dans ce cas le carbonate mis en oeuvre précédemment comme groupe protecteur des fonctions phénol de l'acide caféique ne présente pas une solubilité suffisante à la très basse température de réaction utilisée, environ -40°C.
Dans le cas des dérivés 1,4-O-dicaféoyl, on pratique l'estérification sélective en position 3 avec le trichloroéthylchloroformate en tant que groupe protecteur, puis on utilise directement le produit intermédiaire brut, sans purification par cristallisation, de manière à éviter un réarrangement du groupe 3-carbotrichloroéthoxy en groupe 3,4-O-carbonyl.

Dans un quatrième mode de réalisation relatif à la préparation des dérivés 3-O-caféoyl-4-O-féruoyl, on utilise également l'estérification sélective en position 3 précédente pour préparer le dérivé 3-O-caféoyl de départ.

Dans ces deux derniers modes de réalisation, on effectue le clivage successif des groupes protecteurs et le cas échéant l'hydrolyse des quinides comme indiqué précédemment en liaison avec le second mode de réalisation.

Dans tous les modes de réalisation mis en oeuvre, les composés préparés sont obtenus avec un très bon rendement car chaque étape conduit à une préparation sans isomérisation et les composés peuvent ensuite être purifiés facilement.

Les exemples suivants illustrent l'invention. Dans ceux-ci, les parties et pourcentages sont pondéraux sauf indication contraire.

Les composés de départ et les réactifs proviennent de Fluka AG. Les solvants ont été préalablement rendu anhydres avant usage. On a réalisé la chromatographie liquide moyenne pression (MPLC) sur un équipement Büchi.
Le polyamide mis en oeuvre dans la chromatographie a été préalablement lavé au méthanol, puis séché avant utilisation.

La structure des composés a été vérifiée par résonance magnétique nucléaire du proton (RMN). Les résultats de l'analyse élémentaire, les rendements et les points de fusion sont indiqués.

### Exemples

1. Synthèse de la 3-O-p-coumaroyl quinide et de l'acide 3-O-p-coumaroyl quinique
   **1.1. 3,4-O-Isopropylidène quinide**
      On traite au reflux une suspension de 66,2 g (344,4 mmole) et de 2,1 g (11 mmole) d'acide p-toluènesulfonique dans 900 ml d'acétone pendant 24 h dans un appareil de Soxhlet contenant 90 g de tamis moléculaire de 4 A°. Après refroidissement à O°C, on ajoute 27 g (321,4 mmole) de bicarbonate de sodium. Après agitation du mélange réactionnel 1 h à la température de 0°C, on filtre la suspension et on évapore le solvant. Une recristallisation du résidu dans un mélange dichlorométhane/hexane conduit à 67 g de 3,4-O-isopropylidène quinide de point de fusion (p.f.) 141-142 °C avec un rendement (rdt) de 91%.
   **1.2. 1-O-Carbotrichloroéthoxy-3,4-O-isopropylidène quinide**
      A une solution de 32,1 g (150 mmole) de 3,4-O-isopropylidène quinide et de 28,9 g (365 mmole) de pyridine dans 300 ml de dichlorométhane on ajoute goutte à goutte 34,96 g (165 mmole) de trichloroéthyl chloroformate dilué dans 30 ml de dichlorométhane à 0°C.
      Après 3 h d'agitation à la température ambiante, on lave le mélange réactionnel avec une solution aqueuse d'HCl 2N, puis à l'eau, on sèche la solution sur sulfate de sodium et on évapore les solvants. Une recristallisation du résidu avec l'éthanol conduit à 49,5 g de 1-O-Carbotrichloroéthoxy-3,4-O-isopropylidène quinide (p.f. 165-166°C, rdt 85%).
   **1.3. 1-O-Carbotrichloroéthoxy quinide**
      On agite une solution de 39 g (100 mmole) de 1-O-Carbotrichloroéthoxy-3,4-O-isopropylidène quinide dans 60 ml d'une solution aqueuse à 90% d'acide trichloroacétique pendant 3 h. Après élimination des solvants, on dissout le résidu dans 100 ml d'acétate d'éthyl, on le lave avec un solution aqueuse à 2% de bicarbonate de sodium, puis de la saumure, on le sèche sur sulfate de sodium et on évapore le solvant. Une recristallisation du résidu dans le toluène donne 24,6 g de 1-O-Carbotrichloroéthoxy quinide (p.f. 130-131°C, rdt 70%).
   **1.4. 1-O-Carbotrichloroéthoxy-3-O-carbotrichloroéthoxy-p-coumaroyl quinide**
      On ajoute goutte à goutte à -40°C une solution de 8,95 g (25 mmole) de chlorure d'acide carbotrichloroéthoxy-p-coumarique dans 50 ml de dichlorométhane à une solution de 8,74 g (25 mmole) de 1-O-Carbotrichloroéthoxy quinide et de 2,18 g (27,5 mmole) de pyridine dans 100 ml de dichlorométhane. On agite le mélange réactionnel pendant 24 h à -40°C. Aprés élimination des solvants, on dissout le résidu dans 100 ml d'acétate d'éthyl, puis on lave la solution successivement à l'eau, avec une solution aqueuse d'HCl 0,5N, puis à la saumure, on la sèche sur sulfate de sodium et on élimine les solvants par évaporation. La recristallisation du résidu dans le benzène donne 11,95 g de 1-O-carbotrichloroéthoxy-3-O-carbotrichloroéthoxy-p-coumaroyl quinide (p.f. 154-157°C, rdt 71%).
   **1.5. 3-O-p-coumaroyl quinide**
      On traite 10,74 g (16 mmole) de 1-O-carbotrichloroéthoxy-3-O-carbotrichloroéthoxy-p-coumaroyl quinide dans 40 ml de THF avec 40 ml d'acide acétique et 6,9 g (105 mmole) de poudre de zinc pendant 3 h à la température ambiante sous agitation. Après évaporation des solvants, on ajoute 200 ml d'acétate d'éthyl au résidu et on le traite à 0°C avec une solution aqueuse d'HCl 0,5N. Après séparation de la phase organique, on la lave avec une solution aqueuse d'HCl 0,1N, puis à la saumure et on la sèche sur sulfate de sodium et on évapore le solvant. Une recristallisation du résidu dans l'acétate d'éthyl conduit à 3,97 g de 3-O-p-coumaroyl quinide (p.f. 240,5-242,5°C, rdt 78%), analyse élémentaire: calculé, C=60,00; H=5,04, trouvé, C=59,79; H=5,30.
   **1.6. Acide 3-O-p-coumaroyl quinique**
      On traite au reflux une solution de 2,6 g (8,12 mmole) de 3-O-p-coumaroyl quinide et de 0,08 g (0,41 mmole) d'acide p-toluènesulfonique dans 50 ml de THF aqueux à 50% pendant 24 h. Après élimination des solvants par évaporation, on précipite le résidu avec un mélange de 1 volume de THF pour 10 volumes d'éther. Une recristallisation du'précipité dans le THF donne 1,95 g d'acide 3-O-p-coumaroyl quinique (p.f. 176-178°C, rdt 71%), analyse élémentaire: calculé, C=56,80; H=5,49, trouvé, C=55,54; H=5,43.
2. Synthèse de la 3-O-caféoyl quinide et de l'acide 3-0-caféoyl quinique
   **2.1 1-O-Carbotrichloroéthoxy-3-dicarbométhoxycaféoyl quinide**
      On ajoute goutte à goutte à -47°C une solution de 15,7 g (50 mmole) de chlorure d'acide dicarbométhoxycaféique dans 100 ml de dichlorométhane à une solution de 17,5 g (50 mmole) de 1-O-carbotrichloroéthoxy quinide et de 4,35 g (55 mmole) de pyridine dans 200 ml de dichlorométhane. Après agitation du mélange réactionnel pendant 24 h à -47°C, on élimine les solvants par évaporation, on dissout le résidu dans 230 ml d'acétate d'éthyl, puis on lave la solution successivement avec une solution aqueuse d'HCl 0,5N et à la saumure, on la sèche sur sulfate de sodium et on en évapore les solvants. Une purification du résidu par MPLC sur polyamide avec élution par un mélange de 1 volume de dichlorométhane pour 2 volumes d'hexane donne 24,69 g de 1-0-carbotrichloroéthoxy-3-dicarbométhoxycaféoyl quinide (rdt 79%).
   **2.2 3-O-Dicarbométhoxycaféoyl quinide**
      On traite 16,58g (26,42 mmole) de 1-0-carbotrichloroéthoxy-3-dicarbométhoxycaféoyl quinide dans 30 ml de THF avec 60 ml d'acide acétique et 10,7 g (163,7 mmole) de poudre de zinc pendant 2 h à la température ambiante sous agitation. On élimine le résidu par filtration, puis on évapore le solvant du filtrat. Après dissolution du résidu dans 100 ml d'acétate d'éthyl, lavage de la solution avec une solution aqueuse d'HCl 1N, puis à la saumure, on la sèche sur sulfate de sodium et on en évapore le solvant. Une reprise du résidu huileux par un mélange de chloroforme/hexane conduit à 11,53 g de 3-0-dicarbométhoxycaféoyl quinide sous forme d'un solide amorphe (rdt 97%).
   **2.3. 3-O-caféoyl quinide**
      On traite au reflux un mélange de 11,53 g (26,42 mmole) de 3-O-dicarbométhoxycaféoyl quinide et de 11,4 g (104,9 mmole) de chlorotriméthylsilane dans 75 ml de pyridine pendant 1 h. On ajoute ensuite 12 g (282,3 mmole) de LiCl et on continue le traitement au reflux pendant 2h. Après élimination des solvants par évaporation, on ajoute 100 ml d'acétate d'éthyl au résidu, puis on lave la solution successivement avec une solution aqueuse d'HCl 1N, à l'eau et à la saumure, on la sèche sur sulfate de sodium et on en évapore le solvant. Une recristallisation du résidu par l'acétate d'éthyl conduit à 5,35 g de 3-O-caféoyl quinide (p.f. 225-229°C avec décomposition, rdt 62%), analyse élémentaire: calculé, C=57,14; H=4,80, trouvé, C=56,58; H=4,93.
   **2.4. 1,4-O-Dicarbotrichloroéthoxy-3-O-dicarbotrichloroéthoxycaféoyl quinide**
      On ajoute goutte à goutte 5,33 g (66 mmole) de pyridine et de 14 g (66 mmole) de trichloroéthyl chloroformate à une suspension de 5,1 g (15,2 mmole) de 3-O-caféoyl quinide dans 90 ml de dichlorométhane. Aprés agitation 24 h à la température ambiante, on élimine les solvants par évaporation, on ajoute 100 ml d'acétate d'éthyl au résidu, puis on lave la solution avec une solution aqueuse d'HCl 0,5N et à la saumure, on la sèche sur sulfate de sodium et on évapore le solvant. Une recristallisation du résidu huileux par un mélange de 1 volume de dichlorméthane pour 3 volumes d'éthanol conduit à 10 g de 1,4-O-Dicarbotrichloroéthoxy-3-O-dicarbotrichloroéthoxycaféoyl quinide (rdt 63%).
   **2.5. Acide 1,4-O-dicarbotrichloroéthoxy-3-O-dicarbotrichloroéthoxycaféoyl quinique**
      On agite pendant 5 j à la température ambiante un mélange de 10 g (9,6 mmole) de 1,4-O-dicarbotrichloroéthoxy-3-O-dicarbotrichloroéthoxycaféoyl quinide dans 65 ml de THF et de 8 ml d'HCl concentré. Après séparation du THF et saturation par NaCl, on extrait la phase aqueuse par 3x40 ml d'acétate d'éthyl. On lave ensuite les extraits combinés à la saumure, on les sèche sur sulfate de sodium et on évapore le solvant. Un traitement du résidu par un mélange de 1 volume de dichlorométhane pour 10 volumes d'hexane conduit à 9,5 g d'acide 1,4-O-dicarbotrichloroéthoxy-3-O-dicarbotrichloroéthoxy- caféoyl quinique (rdt 93%) sous forme d'un solide amorphe.
   **2.6. Acide 3-O-caféoyl quinique**
      On traite 1 g (0,94 mmole) d'acide 1,4-O-dicarbotrichloroéthoxy-3-O-dicarbotrichloroéthoxycaféoyl quinique dans 5 ml de THF et 5 ml d'acide acétique avec 2,4 g (37,85 mmole) de poudre de zinc pendant 2 h. Après filtration du précipité, on concentre le filtrat jusqu'à dessiccation. On dissout alors le résidu dans 30 ml d'acétate d'éthyl, on le lave à l'acide sulfurique 2N froid saturé par NaCl, puis à la saumure, on sèche la solution sur sulfate de sodium et on en évapore le solvant. Un traitement du résidu avec un mélange THF/éther/hexane dans les proportions 1:10:10 en volume suivi d'une lyophilisation du précipité donne 0,24 g d'acide 3-O-caféoyl quinique (rdt 73%), analyse élémentaire: calculé, C=54,24; H=5,12, trouvé, C=53,60; H=5,00.
3. Synthèse de la 4-O-p-coumaroyl quinide et de l'acide 4-O-p-coumaroyl quinique
   3.1. En procédant de manière analogue à celle indiquée au paragraphe 1.4 précédent, on prépare la 1,3-O-Dicarbotrichloroéthoxy quinide (p.f. 127-130°C, rdt 67%) par estérification de la 1-O-carbotrichloroéthoxy quinide avec le trichloroéthyl chloroformate.
   **3.2. 1,3-O-Dicarbotrichloroéthoxy-4-O-carbotrichloroéthoxy-p-coumaroyl quinide**
      On ajoute à 0°C 3,58 g (10 mmole) de chlorure d'acide carbotrichloroéthoxy-p-coumarique à une solution de 5,25 g (10 mmole) de 1,3-O-dicarbotrichloroéthoxy quinide et de 0,8 g (10 mmole) de pyridine dans 25 ml de dichlorométhane. Après agitation 24 h à la température ambiante, on élimine les solvants par évaporation. On ajoute alors 70 ml d'acétate d'éthyl au résidu, on lave la solution avec une solution aqueuse d'HCl 1N et à la saumure, on la sèche sur sulfate de sodium et on en évapore le solvant. Une recristallisation du résidu par un mélange solvant dichlorométhane/éther/hexane en proportions volumiques 1:5:5 conduit à 5,65 g de 1,3-O-dicarbotrichloroéthoxy-4-O-carbotrichloroéthoxy-p-coumaroyl quinide (rdt 67%).
   3.3 En procédant de manière analogue à celle indiquée au paragraphe 1.5 précédent, on obtient la **4-O-p-coumaroyl quinide** (rdt 81%), analyse élémentaire: calculé, C=60,00; H=5,04, trouvé, C=59,83; H=5,18, à partir de la 1,3-O-dicarbotrichloroéthoxy-4-O-carbotrichloroéthoxy-p-coumaroyl quinide.
   3.4. En procédant de manière analogue à celle indiquée au paragraphe 2.5 précédent, on prépare l'acide **1,3-O-dicarbotrichloroéthoxy-4-O-carbotrichloroéthoxy-p-coumaroyl quinique** (rdt 94%) à partir de la 1,3-O-dicarbotrichloroéthoxy-4-O-carbotrichloroéthoxy-p-coumaroyl quinide.
   **3.5. Acide 4-O-p-coumaroyl quinique**
      On traite une solution de 5,62 g (6,5 mmole) d'acide 1,3-O-dicarbotrichloroéthoxy-4-O-carbotrichloroéthoxy-p-coumaroyl quinique dans 15 ml de THF et 65 ml d'acide acétique par 12 g (189,2 mmole) de poudre de zinc pendant 3 h à la température ambiante. Après filtration du précipité, on concentre le filtrat jusqu'à dessication. On ajoute alors 60 ml d'acétate d'éthyl au résidu, on lave la solution avec une solution aqueuse d'HCl 1N, puis à la saumure, on la sèche sur sulfate de sodium et on en évapore le solvant. Une recristallisation du résidu dans l'eau conduit à 1,47 g d'acide 4-O-p-coumaroyl quinique (rdt 67%), analyse élémentaire: calculé, C=56,80; H=5,36, trouvé, C=56,74; H=5,39.
4. Synthèse de la 1,3-O-dicaféoyl quinide et de l'acide 1,3-O-dicaféoyl quinique
   **4.1. 1-O-Caféoyl-3,4-O-isopropylidène quinide**
      A une solution de 32,1 g (150 mmole) de 3,4-O-isopropylidène quinide dans 400 ml de dichlorométhane, on ajoute successivement 37,1 g (165 mmole) de chlorure d'acide carbonyl caféique et 13,1 g (165 mmole) de pyridine à 0°C. Après agitation pendant 24 h, on lave le mélange réactionnel avec une solution aqueuse d'HCl 1N, puis à l'eau, on le sèche sur sulfate de sodium et on en évapore les solvants. Après dissolution du résidu dans 300 ml de THF aqueux à 60%, on traite le résidu au reflux pendant 3 h. Après évaporation des solvants sous vide, trituration avec le toluène et recristallisation du résidu dans l'acétate d'éthyl, on obtient 36,3 g de 1-O-Caféoyl-3,4-O-isopropylidène quinide (rdt 64%).
   **4.2. 1-O-dicarbotrichloroéthoxycaféoyl-3,4-O -isopropylidène quinide**
      On ajoute à une solution de 36,2 g (96,2 mmole) de 1-O-Caféoyl-3,4-O-isopropylidène quinide et de 16,7 g (212 mmole) de pyridine dans 250 ml de dichlorométhane 44,9 g (212 mmole) de trichloroéthyl chloroformate dilué dans 30 ml de dichlorométhane à 0°C. Après agitation 1 h à 0°C, puis 1 h à la température ambiante, on lave le mélange réactionnel avec une solution aqueuse d'HCl 1N, puis à l'eau, on le sèche sur sulfate de sodium et on en évapore les solvants. Un lavage du résidu avec 400 ml d'un mélange acétate d'éthyl/hexane dans des proportions volumiques 1:20 donne 67,5 g de 1-O-dicarbotrichloroéthoxycaféoyl-3,4-O-isopropylidène quinide (rdt 97%).
   **4.3. 1-O-Dicarbotrichloroéthoxycaféoyl quinide**
      On agite pendant 5 h une solution de 59 g (81 mmole) de 1-O-dicarbotrichloroéthoxycaféoyl-3,4-O-isopropylidène quinide dans 500 ml d'acide trifluoroacétique aqueux à 90%. Après évaporation des solvants, on ajoute au résidu 200 ml d'acétate d'éthyl, on lave la solution à la saumure, on sèche sur sulfate de sodium et on évapore le solvant. Une chromatographie flash du résidu sur polyamide avec élution par un mélange équivolumique de dichlorométhane/hexane donne 42,9 g de 1-O-dicarbotrichloroéthoxycaféoyl quinide (rdt 77%).
   **4.4. 1-O-Dicarbotrichloroéthoxycaféoyl-3-O -dicarbométhoxycaféoyl quinide**
      A une solution de 17,2 g (25 mmole) de 1-O -dicarbotrichloroéthoxycaféoyl quinide dans 200 ml de dichlorométhane, on ajoute à -45°C 1,98 g (25 mmole) de pyridine , puis goutte à goutte 7,85 g (25 mmole) de chlorure d'acide dicarbométhoxy caféique dilué dans 20 ml de dichlorométhane. Après agitation 24 h à -45°C, on élimine les solvants par évaporation sous vide. On ajoute alors au résidu 250 ml d'acétate d'éthyl, on lave la solution avec une solution aqueuse froide d'HCl 1N, puis à la saumure, on la sèche sur sulfate de sodium et on en évapore le solvant. Une chromatographie flash du résidu sur polyamide avec élution par un mélange dichlorométhane/hexane en proportions volumiques 1:2 donne 15,1 g de 1-O-dicarbotrichloroéthoxycaféoyl-3-O -dicarbométhoxycaféoyl quinide (rdt 63%).
   **4.5. 1,3-O-Dicaféoyl quinide**
      On traite au reflux un mélange de 13,7 g (14,2 mmole) de 1-O-dicarbotrichloroéthoxycaféoyl-3-O -dicarbométhoxycaféoyl quinide et de 15,4 g (132 mmole) de chlorotriméthylsilane dans 140 ml de pyridine pendant 1 h. On ajoute ensuite 12,3 g (284 mmole) de LiCl et on poursuit le reflux pendant 30 min. Après élimination des solvants, on ajoute au résidu 200 ml d'acétate d'éthyl, on lave la solution avec une solution aqueuse d'HCl 1N, puis à la saumure, on la sèche sur sulfate de sodium et on en évapore le solvant. Une recristallisation du résidu dans l'acétate d'éthyl conduit à 5 g de 1,3-O-dicaféoyl quinide (p.f. 185-188°C, rdt 71%), analyse élémentaire: calculé, C=60,24; H=4,45, trouvé, C=60,43; H=4,32.
      En procédant d'une manière analogue à celle indiquée aux paragraphes 2.4-2.6 précédents, on prépare ensuite successivement
   **4.6, la 1,3-O-bis-dicarbotrichloroéthoxycaféoyl-4-O -carbotrichloroéthoxy quinide** (rdt 66%), par estérification de la 1,3-O-dicaféoyl quinide avec le trichloroéthyl carbonate, mise à part une recristallisation du résidu dans l'éthanol (voir 2.4),
   **4.7, l'acide 1,3-bis-dicarbotrichloroéthoxycaféoyl-4-O -carbotrichloroéthoxy quinique** (rdt 92%), mis à part qu'après traitement par l'HCl concentré pendant 2 j., on dilue le mélange réactionnel avec de l'eau, puis on le traite à 0°C avec du bicarbonate de sodium solide jusqu'à pH 4,8, qu'on le sature avec NaCl et qu'on extrait la phase aqueuse avec 3x10 ml de THF au lieu de l'acétate d'éthyl et que l'on obtient le produit désiré en traitant le résidu final avec un mélange THF/hexane en proportions volumiques 1:25 (voir 2.5) et
   **4.8, l'acide 1,3-O-dicaféoyl quinique** (p.f. 227,5-228,5°C, rdt 60%), analyse élémentaire: calculé, C=58,14; H=4,68, trouvé, C=58,14; H=4,72, mis à part la recristallisation du résidu final avec l'acide acétique aqueux à 5% (voir 2.6).
5. Synthèse de la 1,4-O-dicaféoyl quinide et de l'acide 1,4-O-dicaféoyl quinique
   En procédant d'une manière analogue à celle indiquée au paragraphe 4.4 précédent, on prépare
   **5.1, la 1-O-dicarbotrichloroéthoxycaféoyl-3-O -carbotrichloroéthoxy quinide** (obtenue en mélange de 23,3 g contenant 20% de la 1-O-dicarbotrichloroéthoxycaféoyl quinide de départ, comme celà ressort de l'analyse spectroscopique RMN et par chromatographie en couche mince) par réaction de 6,36 g (30 mmole) de trichloroéthyl chloroformate avec 20,61 g (30 mmole) de 1-O-dicarbotrichloroéthoxycaféoyl quinide.
   **5.2. 4-O-Caféoyl-3-O-carbotrichloréthoxy-1-O -dicarbotrichloroéthoxycaféoyl quinide**
      On traite 22 g du mélange solide du paragraphe 8.1 précédent dans 200 ml de dichlorométhane en ajoutant successivement à -10°C 8,2 g (36,5 mmole) de chlorure d'acide carbonyl caféique et 3,6 g (45 mmole) de pyridine. Après agitation 24 h à -10°C, on élimine les solvants par évaporation. On ajoute alors 200 ml d'acétate d'éthyl au résidu, on lave la solution avec une solution aqueuse d'HCl 0,5N, puis à la saumure et on évapore le solvant sous vide. On dissout ensuite le résidu dans 100 ml de THF aqueux à 20% et on traite la solution au reflux pendant 90 min. Après concentration jusqu'à dessiccation, on ajoute 200 ml d'acétate d'éthyl, on lave la solution à la saumure, on la sèche sur sulfate de sodium et on en élimine le solvant par évaporation. Une MPLC du résidu sur polyamide avec élution par un mélange dichlorométhane/hexane dans les proportions volumiques 1:2 conduit à 11,5 g de 4-O-Caféoyl-3-O -carbotrichloroéthoxy-1-O-dicarbotrichloroéthoxycaféoyl quinide (rdt 38%, pour les 2 étapes 5.1 et 5.2).
   **5.3. 1,4-O-Dicaféoyl quinide**
      On traite une solution de 4,1 g (4 mmole) de 4-O-Caféoyl-3-O-carbotrichloroéthoxy-1-O -dicarbotrichloroéthoxycaféoyl quinide dans 10 ml de THF et 15 ml d'acide acétique avec 5,07 g (80 mmole) de poudre de zinc pendant 3 h. Après élimination des solvants, on ajoute 20 ml d'acétate d'éthyl au résidu, on lave la solution avec une solution froide d'HCl 0,5N, puis à la saumure, on la sèche sur sulfate de sodium et on en évapore le solvant. Un traitement du produit brut par un mélange solvant acétate d'éthyl/chloroforme en proportions volumiques 1:10 suivi de la lyophilisation du résidu conduit à 1,38 g de 1,4-Dicaféoyl quinide (rdt 69%), analyse élémentaire: calculé, C=60,24; H=4,45, trouvé, C=60,56; H=4,25.
   **5.4. Acide 4-O-caféoyl-3-O-carbotrichloroéthoxy-1-O dicarbotrichloroéthoxycaféoyl quinique**
      On traite une solution de 7 g (6,83 mmole) de 4-O-Caféoyl-3-O-carbotrichloroéthoxy-1-O -dicarbotrichloroéthoxycaféoyl quinide dans 30 ml de THF avec 5 ml d'HCl concentré pendant 3 j. Après dilution du mélange réactionnel avec 10 ml d'eau, on le traite avec du bicarbonate de sodium jusqu'à pH 4 à 0°C. Après l'avoir saturée au NaCl, on sépare la phase organique. On extrait alors la phase aqueuse avec 3x15 ml d'acétate d'éthyl. On lave ensuite les phases organiques combinées à la saumure, on les sèche sur sulfate de sodium et on en évapore les solvants. Une recristallisation du résidu par un mélange solvant THF/éther/hexane en proportions volumétriques 1:5:20 conduit à 6,28 g d'acide 4-O-caféoyl-3-O-carbotrichloroéthoxy-1-O-dicarbotrichloroéthoxycaféoyl quinique (rdt 88%).
   **5.5 Acide 1,4-O-dicaféoyl quinique**
      On traite une solution de 2,09 g (2 mmole) d'acide 4-O-caféoyl-3-O-carbotrichloroéthoxy-1-O-dicarbotrichloroéthoxycaféoyl quinique dans 10 ml de THF et 10 ml d'acide acétique avec 2,5 g (40 mmole) de poudre de zinc pendant 2 h. Après élimination des solvants, on ajoute 50 ml d'acétate d'éthyl au résidu, puis on le traite avec une solution aqueuse 2N d'acide sulfurique à 0°C. Après saturation avec NaCl,on sépare l'acétate d'éthyl, puis on extrait la phase aqueuse avec 3x10 ml d'acétate d'éthyl. On lave ensuite les phases organiques combinées avec une solution aqueuse froide d'acide sulfurique 2N, puis à la saumure, on les sèche sur sulfate de sodium et on en évapore le solvant. Une MPLC du résidu sur silicagel avec élution par un mélange solvant n-butanol/éthanol/eau en proportions volumiques 6:1:0,5, suivie d'un lyophilisation donne 0,74 g d'un mélange contenant 95% d'acide 1,4-O-dicaféoyl quinique et 5% d'acide 1,3-O-dicaféoyl quinique (rdt 72%), comme on l'a vérifié par spectroscopie RMN.
6. Synthèse de la 3-O-caféoyl-4-O-féruloyl quinide et de l'acide 3-O-caféoyl-4-O-féruloyl quinique
   **6.1. 1-O-Carbotrichloroéthoxy-4-O -carbotrichloroéthoxyféruloyl-3-O-dicarbométhoxycaféoyl quinide**
      A une solution de 15,7 g (25 mmole) de 1-O-carbotrichloroéthoxy-3-O-dicarbométhoxycaféoyl quinide dans 150 ml de dichlorométhane, on ajoute à -10°C, successivement, 10,7 g (27,2 mmole) de chlorure d'acide carbotrichloroéthoxy férulique et 2,17 g (27,2 mmole) de pyridine. Après agitation 24 h à 0°C, on lave le mélange réactionnel avec une solution aqueuse d'HCl 1N, puis à l'eau, on le sèche sur sulfate de sodium et on en évapore les solvants. Une MPLC du résidu sur polyamide avec élution par un mélange de solvants dichlorométhane/hexane en proportions volumiques 1:4, puis une recristallisation dans un mélange des mêmes solvants en proportions volumiques 1:20 donne 14,4 g de 1-O-Carbotrichloroéthoxy-4-O -carbotrichloroéthoxyféruloyl-3-O-dicarbométhoxycaféoyl quinide (rdt 60%).
   **6.2. 3-O-Dicarbométhoxycaféoyl-4-O-féruloyl quinide**
      On traite une solution de 5,6 g (5,7 mmole) de 1-O-Carbotrichloroéthoxy-4-O -carbotrichloroéthoxyféruloyl-3-O-dicarbométhoxycaféoyl quinide dans 55 ml de THF et 55 ml d'acide acétique avec 3,7 g (57,4 mmole) de poudre de zinc pendant 2 h. Après élimination des solvants, on ajoute 50 ml de dichlorométhane, on lave la solution avec une solution aqueuse d'HCl 0,5N, puis à l'eau, on la sèche sur sulfate de sodium et on en évapore le solvant. Un traitement du résidu avec un mélange solvant dichlorométhane/hexane en proportions volumiques 1:10 donne 3,22 g de 3-O-dicarbométhoxycaféoyl-4-O-féruloyl quinide (rdt 90%).
   **6.3. 3-O-Caféoyl-4-O-féruloyl quinide**
      On traite au reflux une suspension de 4,5 g (106 mmole) de LiCl et de 2,74 g (4,4 mmole) de 3-O-dicarbométhoxycaféoyl-4-O-féruloyl quinide dans 30 ml de pyridine pendant 1 h. Après élimination du solvant, on ajoute 100 ml d'acétate d'éthyl au résidu, on lave la solution avec une solution aqueuse d'HCl 0,5N, puis à la saumure, on la sèche sur sulfate de sodium et on en évapore le solvant. Un traitement du résidu avec un mélange solvant dichlorométhane/hexane en proportions volumiques 1:10 donne 1,44 g de 3-O-caféoyl-4-O-féruloyl quinide (rdt 64%), analyse élémentaire: calculé, C=60,94; H=4,72, trouvé C=61,10; H=4,48.
   **6.4. Acide 3-O-caféoyl-4-O-féruloyl quinique**
      On traite au reflux une solution de 0,84 g (1,64 mmole) de 3-O-caféoyl-4-O-féruloyl quinide dans 20 ml d'acétonitrile aqueux à 50% contenant 10 gouttes d'acide trifluoroacétique pendant 24 h. Après élimination du solvant, on ajoute 50 ml d'acétate d'éthyl au résidu, on lave la solution à la saumure, on la sèche sur sulfate de sodium et on en élimine le solvant par évaporation.
      Un traitement du résidu par un mélange de solvants acétate d'éthyl/hexane en proportions volumiques 1:5 suivi d'une lyophilisation donne 0,44 g d'acide 3-O-caféoyl-4-O -féruloyl quinique (rdt 51%), analyse élémentaire: calculé, C=58,87; H=4,94, trouvé, C=59,07; H=4,96.

Les exemples 2.1 à 2.6 ne font plus partie du domaine revendiqué mais servent d'illustration à la synthèse des composés nouveaux des exemples 4.6 à 4.8.

## Revendications

1. Quinides, destinées à servir de composés intermédiaires dans la synthèse d'acides quiniques 3- ou 4-mono-, 1,3-, 1,4- ou 3,4-disubstitués, de formule 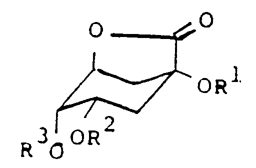 dans laquelle R1 est H et l'un des deux groupes R2 et R3 représente un groupe p-coumaroyl et l'autre est H, ou les deux groupes R2 et R3 représentent un groupe p-coumaroyl, p-feruloyl ou p-caféoyl, R2 étant différent de R3, ou R1 est un groupe p-coumaroyl, p-feruloyl ou p-caféoyl et R2 est H et R3 représente un groupe p-coumaroyl ou p-feruloyl ou R2 est un groupe p-coumaroyl, p-feruloyl ou p-caféoyl et R3 est H.

2. Procédé de préparation d'acide quinique 3- ou 4-mono-, 1,3-, 1,4- ou 3,4-disubstitué, dans lequel le substituant en position 3 est différent du substituant en position 4 et dans lequel on fait réagir un dérivé d'un acide hydroxycinnamique dont le ou les groupes hydroxyles sont protégés, avec un dérivé de l'acide quinique protégé, de manière à former un ester, puis on clive les groupes protecteurs, caractérisé par le fait que le dérivé de l'acide quinique mis en oeuvre est une quinide et que le dérivé d'acide quinique final est obtenu par hydrolyse de la quinide dans des conditions ménagées, que l'on protège les fonctions OH en position 3, 4 et 5 et la fonction carboxylique en position 1 de l'acide quinique par réaction avec l'acétone conduisant à la 3,4-O-isopropylidène quinide, puis on introduit un groupe protecteur carbonate en position 1, que l'on libère les fonctions OH en positions 3 et 4, que l'on estérifie sélectivement la position 3 en conduisant l'estérification à une température inférieure à - 10°C avec un dérivé réactif d'un acide hydroxycinnamique dont la ou les fonctions phénol ont été préalablement protégées par des groupes carbonates, que l'on libère le groupe protecteur acétonide, puis le ou les groupes protecteurs carbonates dans des conditions ménagées d'acidité et de température, les dites conditions ménagées permettant d'éviter toute dégradation ou isomérisation lors de la déprotection.

3. Procédé selon la revendication 2, de préparation de dérivés monosubstitués 3-O-p-coumaroyl ou 3-O-féruloyl, caractérisé par le fait que le groupe protecteur de la fonction phénol est le trichloroéthyl carbonate, que l'on libère le groupe acétonide protecteur des positions 3 et 4 par l'acide trifluoroacétique aqueux à la température ambiante, puis que l'on clive le trichloroéthyl carbonate par le zinc en milieu acétique à la température ambiante.

4. Procédé selon la revendication 2, de préparation de dérivés 3-O-caféoyl, caractérisé par le fait que le groupe protecteur des fonctions phénol est le méthyl carbonate, que l'on libère le groupe acétonide protecteur des positions 3 et 4 par l'acide trifluoroacétique aqueux à la température ambiante, et que l'on libère successivement le groupe protecteur trichloroéthyl carbonate par le zinc en présence d'acide acétique, puis les groupes méthyl carbonate par le chlorure de lithium en présence de chlorotriméthylsilane dans la pyridine.

5. Procédé selon la revendication 2, de préparation de dérivés monosubstitués 4-O-hydroxycinnamoyl, caractérise par le fait que l'on protège les fonctions 1 et 3 d'une quinide par des groupes trichloroéthyl carbonate, puis que l'on estérifie la position 4 par un dérivé réactif d'un acide hydroxycinnamoyl dont la ou les fonctions phénol on été préalablement protégées, puis que l'on clive les groupes protecteurs des positions 1 et 3.

6. Procédé selon la revendication 2, de préparation de dérivés 1,3-O- et 1,4-O-disubstitués, caractérisé par le fait que, les positions 3 et 4 ayant été protégées, on estérifie la position 1 par un dérivé réactif d'un acide hydroxycinnamoyl protégé, on libère les positions 3 et 4, puis on estérifie la position 3 par un dérivé réactif d'un acide hydroxycinnamoyl protégé dans le cas de la préparation des dérivés 1,3-O-disubstitués, et dans le cas de la préparation de dérivés 1,4-O-disubstitués, on protège la position 3 par un carbonate préalablement à l'estérification en position 4 par un dérivé réactif d'un acide hydroxycinnamique protégé.

7. Procédé selon la revendication 2, de préparation de dérivés 3,4-O-disubstitués, caractérisé par le fait qu'après avoir protégé la position 1 de la quinide, on estérifie les positions 3, puis 4 par un dérivé réactif d'un acide hydroxycinnamique dont les fonctions phénol ont été préalablement protégées.

8. Procédé selon l'une des revendications 2 à 7, caractérisé par le fait que l'on transforme la quinide en acide quinique par hydrolyse acide ménagée par l'acide chlorhydrique concentré à basse température en présence d'un solvant polaire.

9. Procédé selon la revendication 8, de préparation d'un acide caféoyl quinique, caractérisé par le fait que l'on réalise l'hydrolyse du cycle lactone de la quinide entièrement protégée préalablement au clivage des groupes protecteurs.

## Claims

1. Quinides, intended to act as intermediate compounds in the synthesis of 3- or 4-mono-, 1,3-, 1,4- or 3,4-disubstituted quinic acids, having the formula 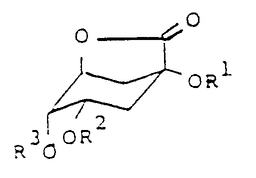 in which R1 is H and one of the two groups R2 and R3 represent a p-coumaroyl group and the other is H, or the two groups R2 and R3 represent a p-coumaroyl, p-feruloyl or p-cafeoyl group, R2 being different from R3, or R1 is a p-coumaroyl, p-feruloyl or p-cafeoyl group and R2 is H and R3 represents a p-coumaroyl or p-feruloyl group or R2 is a p-coumaroyl, p-feruloyl or p-cafeoyl group and R3 is H.

2. Method for preparing 3- or 4-mono, 1,3-, 1,4- or 3,4-disubstituted quinic acid in which the substituent in the 3 position is different from the substituent in the 4 position and in that a derivative of a hydroxycinnamic acid of which the hydroxyl group or groups is/are protected is made to react with a protected quinic acid derivative so as to form an ester, and the protective groups are then split off, characterized in that the quinic acid derivative used is a quinide and that the final quinic acid derivative is obtained by hydrolysis of the quinide under controlled conditions, that the OH functional groups in the 3, 4 and 5 positions are protected and the carboxylic functional group in the 1 position of quinic acid is protected by reaction with acetone leading to 3,4-O-isopropylidene quinide, a carbonate protective group is then introduced in the 1 position, that the OH functional groups in positions 3 and 4 are liberated, that the 3 position is selectively esterified by carrying out esterification at a temperature below -10°C with a reactive derivative of a hydroxycinnamic acid of which the phenol functional group or groups has/have been previously protected by carbonate groups, that the protective acetonide group is liberated, followed by the protective carbonate group or groups under controlled conditions of acidity and temperature, the said controlled conditions making it possible to prevent any degradation or isomerisation during deprotection.

3. Method according to claim 2, for preparing 3-O-p-coumaroyl or 3-O-feruloyl monosubstituted derivatives, characterized in that the group protecting the phenol functional group is trichloroethyl carbonate, that the acetonide group protecting the 3 and 4 positions is liberated with aqueous trifluoroacetic acid at room temperature, and that the trichloroethyl carbonate is then split off by zinc in an acetic medium at room temperature.

4. Method according to claim 2, for preparing 3-O-cafeoyl derivatives, characterized in that the group protecting the phenol functional groups is methyl carbonate, that the acetonide group protecting the 3 and 4 positions is liberated by aqueous trifluoroacetic acid at room temperature,
and that there are liberated successively the trichloroethyl carbonate protective group by zinc in the presence of acetic acid, followed by methyl carbonate groups by lithium chloride in the presence of chlorotrimethylsilane in pyridine.

5. Method according to claim 2, for preparing 4-O-hydroxycinnamoyl monosubstituted derivatives, characterized in that the 1 and 3 functional groups of a quinide are protected by trichloroethyl carbonate groups, the 4 position is then esterified with a reactive derivative of a hydroxycinnamoyl acid of which the phenol functional group or groups has/have been previously protected, and the groups protecting the 1 and 3 positions are then split off.

6. Method according to claim 2, for preparing 1,3-O- and 1-4-O-disubstituted derivatives, characterized in that the 3 and 4 positions having been protected, the 1 position is esterified with a reactive derivative of a protected hydroxycinnamoyl acid, the 3 and 4 positions are liberated, the 3 position is then esterified with a reactive derivative of a protected hydroxycinnamoyl acid in the case of the preparation of 1,3-O-disubstituted derivatives, and in the case of the preparation of 1,4-O-disubstituted derivatives, the 3 position is protected by a carbonate prior to the esterification in the 4 position with a reactive derivative of a protected hydroxycinnamic acid.

7. Method according to claim 2, for preparing 3,4-O-disubstituted derivatives, characterized in that after having protected the 1 position of the quinide, the 3 position, followed by the 4 position is esterified with a reactive derivative of a hydroxycinnamic acid of which the phenol functional group or groups has/have been previously protected.

8. Method according to one of claims 2 to 7, characterized in that the quinide is converted into quinic acid by controlled acid hydrolysis with concentrated hydrochloric acid at a low temperature in the presence of a polar solvent.

9. Method according to claim 8, for preparing a cafeoyl quinic acid, characterized in that the lactone ring of the entirely protected quinide is hydrolysed prior to splitting off the protective groups.

## Patentansprüche

1. Chinide, die bestimmt sind, als Zwischenstufen bei der Synthese von 3- oder 4-mono-, 1,3-, 1,4- oder 3,4-di-substituierten Chinasauren zu dienen, mit der Formel 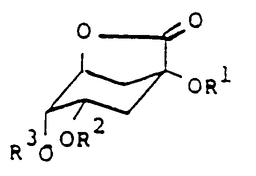 bei der R¹ H ist und eine der beiden Gruppen R² und R³ für eine p-Cumaroylgruppe steht und die andere H ist, oder beide Gruppen R² und R³ für eine p-Cumaroyl-, p-Feruloyl- oder p-Caffeoyl-Gruppe stehen, wobei sich R² von R³ unterscheidet, oder bei der R¹ eine p-Cumaroyl, p-Feruloyl- oder p-Caffeoyl-Gruppe ist und R² H ist und R³ für eine p-Cumaroyl- oder p-Feruloyl-Gruppe steht oder R² eine p-Cumaroyl-, p-Feruloyl- oder p-Caffeoyl-Gruppe ist und R³ H ist.

2. Verfahren zur Herstellung von 3- oder 4-mono-, 1,3-, 1,4- oder 3,4-disubstituierter Chinasäure, bei der sich der Substituent in der Position 3 von dem Substituenten in der Position 4 unterscheidet, bei dem man ein Derivat einer Hydroxyzimtsäure, deren Hydroxylgruppe(n) geschützt sind, mit einem geschützten Chinasäurederivat umsetzt, und zwar so, daß ein Ester gebildet wird, wonach man die Schutzgruppen abspaltet, dadurch gekennzeichnet, daß das eingesetzte Chinasäurederivat ein Chinid ist und daß man das endgültige Chinasäurederivat durch Hydrolyse des Chinids bei schonenden Bedingungen gewinnt, daß man die OH-Funktionen in Position 3, 4 und 5 und die Carboxylfunktion in Position 1 der Chinasäure durch Reaktion mit Aceton schützt, die zu einem 3,4-O-Isopropylidenchinid führt, wonach man eine Carbonatschutzgruppe in Position 1 einführt, daß man die OH-Funktionen in den Positionen 3 und 4 freisetzt, daß man Position 3 selektiv verestert und die Veresterung bei einer Temperatur unterhalb von -10°C mit einem reaktiven Hydroxyzimtsäurederivat durchführt, bei dem die phenolische(n) Funktion(en) vorher mit Carbonatgruppen geschützt wurden, daß man die Acetonidschutzgruppe abspaltet, und danach die Carbonatschutzgruppe(n) unter schonenden Säure- und Temperaturbedingungen, wobei es die besagten schonenden Bedingungen gestatten, jeglichen Abbau oder jegliche Isomerisierung bei der Abspaltung der Schutzgruppen zu vermeiden.

3. Verfahren nach Anspruch 2, zur Herstellung von monosubstituierten 3-O-p-Cumaroyl- oder 3-O-Feruloyl-Derivaten, dadurch gekennzeichnet, daß die Schutzgruppe für die phenolische Funktion Trichlorethylcarbonat ist, daß man die Acetonidschutzgruppe der Positionen 3 und 4 mit wässriger Trifluoressigsäure bei Raumtemperatur freisetzt, wonach man das Trichlorethylcarbonat mit Zink im essigsauren Milieu bei Umgebungstemperatur abspaltet.

4. Verfahren nach Anspruch 2, zur Herstellung von 3-O-Caffeoylderivaten, dadurch gekennzeichnet, daß die Schutzgruppe der phenolischen Funktionen Methylcarbonat ist, daß man die Acetonidschutzgruppe der Positionen 3 und 4 mit wässriger Trifluoressigsäure bei Umgebungstemperatur freisetzt, daß man, schrittweise, die Trichlorethylcarbonatschutzgruppe mit Zink in Gegenwart von Essigsäure freisetzt, und danach die Methylcarbonatgruppen mit Lithiumchlorid in Gegenwart von Chlortrimethylsilan in Pyridin.

5. Verfahren nach Anspruch 2, zur Herstellung von monosubstituierten 4-O-Hydroxyzimtsäurederivaten, dadurch gekennzeichnet, daß man die Funktionen 1 und 3 eines Chinids mit Trichlorethylcarbonat-Gruppen schützt, wonach man die Position 4 mit einem reaktiven Derivat einer Hydroxyzimtsäure verestert, deren phenolische Funktion(en) vorher geschützt wurden, wonach man die Schutzgruppen in den Positionen 1 und 3 abspaltet.

6. Verfahren nach Anspruch 2, zur Herstellung von 1,3-O- und 1,4-O-disubstituierten Derivaten, dadurch gekennzeichnet, daß man, während die Positionen 3 und 4 geschützt sind, die Position 1 mit einem reaktiven Derivat einer geschützten Hydroxyzimtsäure verestert, daß man die Positionen 3 und 4 freisetzt, daß man danach die Position 3 mit einem reaktiven geschützten Hydroxyzimtsäurederivat verestert, und zwar im Falle der Herstellung von 1,3-O-disubstituierten Derivaten, und daß man im Falle der Herstellung von 1,4-O-disubstituierten Derivaten die Position 3 vor einer Veresterung der Position 4 mit einem reaktiven Derivat einer geschützten Hydroxyzimtsäure mit einem Carbonat schützt.

7. Verfahren nach Anspruch 2, zur Herstellung von 3,4-O-disubstituierten Derivaten, dadurch gekennzeichnet, daß man, nachdem die Position 1 des Chinids geschützt wurde, die Positionen 3 und danach 4 mit einem reaktiven Derivat einer Hydroxyzimtsäure verestert, deren phenolische Funktionen vorher geschützt wurden.

8. Verfahren nach einem der Ansprüche 2 bis 7, dadurch gekennzeichnet, daß man das Chinid durch eine schonende Säurehydrolyse mit konzentrierter Chlorwasserstoffsäure bei niedriger Temperatur in Gegenwart eines polaren Lösungsmittels in die Chinasäure umwandelt.

9. Verfahren nach Anspruch 8, zur Herstellung einer CaffeoylChinasäure, dadurch gekennzeichnet, daß man die Hydrolyse des vollständig geschützten Lactonrings vor der Abspaltung der Schutzgruppen durchführt.
